# EUROPEAN PATENT SPECIFICATION

(11) **EP 2 427 236 B1**
(45) Date of publication and mention of the grant of the patent: **15.01.2014**
(21) Application number: 10772086.4
(22) Date of filing: 13.06.2010
(51) Int. Cl.: A61M 5/24, A61M 5/172, G08B 21/02, G09F 13/00

(54) **MEDICATION INJECTION SUPERVISOR DEVICE**
VORRICHTUNG ZUR ÜBERWACHUNG MEDIZINISCHER INJEKTIONEN
DISPOSITIF DE SUPERVISION D'INJECTION DE MÉDICAMENT

(30) Priority: 06.05.2009 US 175810 P; 15.03.2010 US 724411
(43) Date of publication of application: 14.03.2012
(73) Proprietor: Hughes, John, Dublin D2 (IE); Cirillo, William, Dublin D2 (IE)
(72) Inventor: Hughes, John, Dublin D2 (IE); Cirillo, William, Dublin D2 (IE)
(74) Representative: Gordon, Naoise Padhraic Edward
(86) International application number: PCT/IB2010/052626
(87) International publication number: WO 2010/128493

(56) References cited:
- EP-A1- 2 060 284
- WO-A1-99/02210
- WO-A1-2006/045525
- WO-A1-2007/107564
- WO-A2-2004/010231
- US-A- 4 529 401
- US-A- 5 728 074
- US-A1- 2004 122 355
- US-B1- 6 277 099
- US-B1- 6 482 185
- US-B1- 6 585 698

## Description

### FIELD

The present invention relates generally to devices for management of chronic medical conditions requiring periodic administration of medications by self injection.
More specifically, the present invention relates to an assembly adaptable to a variety of medication injection delivery devices.

### BACKGROUND

To maintain optimum conditions, a patient suffering from a chronic medical condition is required to conform to a prescribed administration schedule of a medication, adhere to a prescribed dosage, avoid extra administrations, avoid missed administrations, and adhere to various recommended health and safety best practices.

The long-term health of the patient with a chronic medical condition depends on the day-to-day management of the condition. Mismanagement of the condition can result in significant morbidity and mortality and carry an increased risk of developing complications. Focused approach to management of a chronic medical condition is essential for the patient, in order to reduce the occurrence of these complications.

For example, diabetes occurs when the body does not produce enough insulin resulting for many diabetics in a requirement of a periodic insulin injection to control glucose levels in the body. One of the dangers associated with controlling glucose levels with insulin is insulin overdose. Symptoms of an insulin overdose reflect low blood sugar levels (hypoglycemia) and can include headache, irregular heartbeat, increased heart rate or pulse, sweating, tremor, nausea, increased hunger, and anxiety.

An insulin dependent diabetic needs to keep accurate track of the type and amount of insulin he is injecting. Individual insulin products are numerous, but currently insulin may be divided into four major types: 1) Short-acting insulin, which is soluble and acts quickly (within 30-60 minutes) and lasts between 6 and 8 hours. Some subtypes of this soluble insulin may act faster and last for a shorter time. 2) Intermediate-acting insulin - isophane insulin, which acts slightly more slowly (within 1-2 hours) and lasts between 10 and 14 hours. 3) Long-acting insulin such as determir, glargine, protamine zinc, and zinc suspension, which acts comparatively slowly (1-2 hours) and lasts comparatively much longer, for up to 24 hours. 4) Various mixtures of the above- mentioned three major types of insulin. Different amounts of the short and intermediate-acting insulin can be mixed together depending on the requirements of an individual case. A user can suffer long-term health consequences if too little insulin is taken. If, on the other hand, too much insulin is taken, the user can suffer immediate hypoglycemia leading to coma and hospitalization.

WO 2007/107564 A1, EP 2 060 284 A1, WO 99/02210 A1, WO 2006/045525 A1 and WO 2004/010231 A2 all describe similar medication injection supervisor devices for monitoring dosages from injection pens, the supervisor devices being attached to the injection pens in a variety of ways.

### SUMMARY

According to the invention there is provided a medication injection supervisor device comprising a universal header mountable on a prefabricated injection pen, and an electronics assembly housed by the universal header to record time, the medication injection supervisor device being automatically triggerable whenever a user injects medication to provide a reliable means to note the time of or time elapsed since the last injection characterised in that the medication injection supervisor device further comprises a sleeve adapted to the prefabricated injection pen, the universal header being attachable to the sleeve for mounting the medication injection supervisor device on the prefabricated injection pen.

This summary is provided to introduce a selection of concepts in a simplified form that are further described below in the detailed description. This summary is not intended to identify key features or essential features of the claimed subject matter, not is it intended to be used as an aid in determining the scope of the claimed subject matter.

A medication injection supervisor device, in one example embodiment, comprises a particular sleeve, the particular sleeve being designed to rigidly lock onto to a pre-fabricated injection pen of a particular design, a universal header designed to rigidly lock onto the particular sleeve irrespective of the particular model of the prefabricated injection pen, an electronics assembly housed by the universal hender, a sensor to detect an injection automatically, the sensor being communicatively coupled to the electronics assembly, a display to display injection data, and a button to allow manipulation and display of the injection data, including resetting the time. The medication injection supervisor device can infer the completion of the injection from depressing of a plunger of the injection pen.

The medication injection supervisor device can further include a dosage reading system to read a dosage of medication delivered by the prefabrication injection pen, the dosage reading system including a clip specific to the prefabricated injection pen coupled to a plunger of the prefabricated injection pen, the dosage being determined by a position of the clip after an injection. The medication injection supervisor device can further include a micro optics subsystem communicativety coupted to the electronics assembly and having a dial reader to take one or more images of a dosage dial of the prefabricated injection pen, and an imaging subsystem communicatively coupled to the electronics assembly to recognize characters in the images taken by the dial reader.

### BRIEF DESCRIPTION OF DRAWINGS

Example embodiments are illustrated by way of example and not limitation in the figures of the accompanying drawings, in which like references indicate similar elements and in which:
Figure 1 is a perspective view of a medication injection supervisor device, in accordance with an example embodiment;
Figure 2 shows the measurement history of a medication injection supervisor device in accordance with an example embodiment;
Figure 3 shows various messages that may appear on the display sereen of a medication injection supervisor device, in accordance with an example embodiment:
Figure 4 is a perspective view of plunger break or locking pin device of a medication injection supervisor device, in accordance with an example embodiment;
Figure 5 is a perspective view of a medication injection supervisor device mounted on an injection pen, in accordance with an example embodiment;
Figure 6 is an inverted view of a universal header, in accordance with an example embodiment;
Figure 7 is a sleeve specific to an injection pen model, in accordance with an example embodiment;
Figure 8 is a universal header being mounted on a sleeve, in accordance with an example embodiment;
Figure 9 is a universal header mounted on a sleeve, in accordance with an example embodiment;
Figure 10 is an empty universal header and sleeve assembly mounted on an injection pen with locking indents visible, in accordance with an example embodiment;
Figure 11 is an exploded view of an medication injection supervisor device, in accordance with an example embodiment;
Figure 12 shows a sleeve being mounted on a Sanofi Aventis Lantus SoloStar injection pen, in accordance with an example embodiment;
Figure 13 shows a universal header being installed on a sleeve mounted on a Sanofi Aventis Lantus SoloStar injection pen, in accordance with an example embodiment;
Figure 14 shows a sleeve being mounted on a Novo Nordisk FlexPen injection pen, in accordance with an example embodiment;
Figure 15 shows a universal header being installed on a sleeve mounted on a Novo Nordisk FlexPen injection pen, in accordance with an example embodiment;
Figure 16 is a flow diagram showing a method for supervising injections using the medication injection supervising device, in accordance with an example embodiment;
Figure 17 shows a readable plunger subsystem, in accordance with an example embodiment; and
Figure 18 shows a micro optics and imaging system, in accordance with an example embodiment.

### DETAILED DESCRIPTION

The following detailed description includes references to the accompanying drawings, which form a part of the detailed description. The drawings show illustrations in accordance with example embodiments. These example embodiments, which are also referred to herein as "examples," are described in enough detail to enable those skilled in the art to practice the present subject matter. The embodiments can be combined, and other embodiments can be formed by introducing structural, logical or electrical changes without departing from the scope of what is claimed. The following detailed description is, therefore, not to be taken in a limiting sense, and the scope is defined by the appended claims and their equivalents.

In this document, the terms "a" or "an" are used, as is common in patent documents, to include one or more than one. In this document, the term "or" is used to refer to a nonexclusive "or," such that "A or B" includes "A but not B," "B but not A," and "A and B," unless otherwise indicated. Furthermore, all publications, patents, and patent documents referred to in this document are incorporated by reference herein in their entirety, as though individually incorporated by reference. In the event of inconsistent usages between this document and those documents so incorporated by reference, the usage in the incorporated reference(s) should be considered supplementary to that of this document; for irreconcilable inconsistencies, the usage in this document controls.

In the example embodiments described herein, the medication injection supervisor device is an attachment to a standard injection pen, which is sufficiently small to be carried by patients in a pocket or a purse. The medication injection supervisor device can allow patients to keep accurate injection records. In addition to providing a method for keeping one's medication injection records, the medication injection supervisor device can provide reminders and other warnings about events that may affect patient's health.

In some example embodiments, the medication injection supervisor device can record events and generate reports to spot trends and patterns so that adjustments can be made to medication dosage, exercise, or eating. This kind of record keeping can provide someone with a chronic medical condition with a long-term view of his disease that will in turn help him control the disease and lead a healthier life without complications therefrom.

The medication injection supervisor device, according to an example embodiment, can be mounted on a variety of prefabricated injection pens to record the time since last injection. The device can automatically trigger whenever the user injects the medication and thus can provide a reliable means to note the time elapsed since the last injection. Prefabricated injection pens can be of various shapes, depending on the manufacturer. To accommodate these various shapes, a two-part attachment method can be used. The first part can be a sleeve designed to lock solidly and robustly on the particular injection pen. The second part can be a universal header attached to the sleeve unit and housing the electronics.

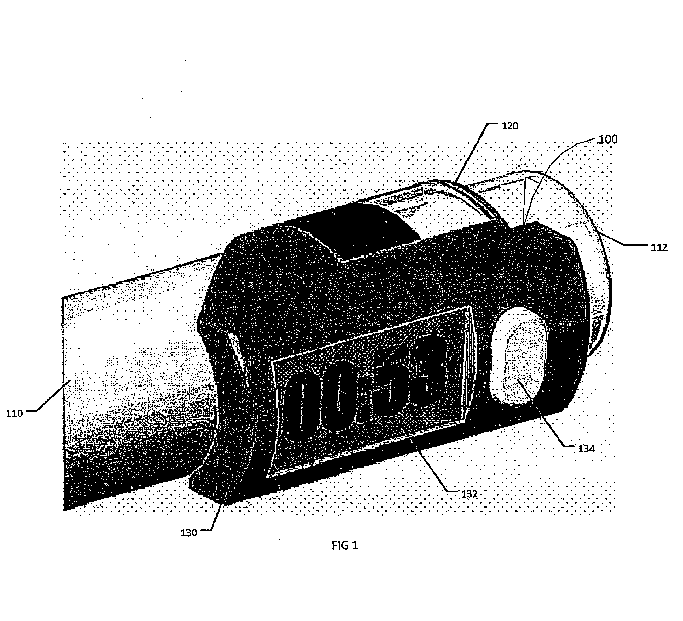

Figure 1 is a perspective view of a medication injection supervisor device 100, in accordance with an example embodiment. As shown in Figure 1, the medication injection supervisor device 100 includes a sleeve 120 and a universal header 130. An injection pen 110 is prefabricated and is not a part of the medication injection supervisor device 100. The injection pen 110 can include a plunger 112, which can be extended to dial a medication dosage. The universal header 130 can include a display 132 to display various data related to medication injection supervision. For example, as shown in Figure 1, the display 132 can display the time since last injection. The universal header 130 can also include one or more buttons 134 to allow the user to manipulate and display the data related to medication injection supervision.

In some example embodiments, the one or more buttons 134 can be used to turn on the display 132. In some example embodiments, it is not necessary to utilize the one or more buttons 134 to turn off the display 132, which turns off automatically after a certain period of time. In some example embodiments, the display 132 does not display the time of day, as the time displayed is elapsed time only. Furthermore, there is no requirement for start and end times of injections to be displayed.

As shown in Figure 1, the sleeve 120 is adapted to the injection pen 110 and the universal header 130 is locked onto the sleeve 120 at locking indents (not shown). This approach allows the universal header 130 to be mounted on a variety of existing injection pens. Thus, the medication injection supervisor device 100 facilitates a two-part attachment method, wherein the sleeve 120 is customized to fit a specific model of an injection pen. This allows the universal header 130 that houses the electronics to be used on a variety of injection pens. Additionally, this allows easy attachment and removal of the medication injection supervisor device 100, and for its precise positioning for various injection pen types without complex steps. In some example embodiments, the medication injection supervisor device 100 is fully automatic and requires no user input so that there is no need to confirm any action and /or to record an event.

The medication injection supervisor device 100 can be characterized as a clip-on injection recorder that attaches to the injection 110 pen to assist in maintaining optimum patient control. The medication injection supervisor device 100 is a clip-on designed to fit an injection pen of a specific brand. The header 130, shown in Figure 1 can be robust enough for regular use and simple enough for very young children and the elderly to fit and remove. The design of the medication injection supervisor device 100 is slim and lightweight so as not to increase the total bulkiness of the injection pen 110 significantly.

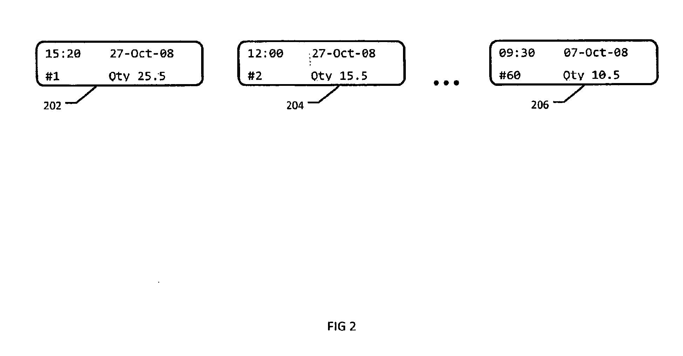

Figure 2 shows the measurement history of the medication injection supervisor device 100, in accordance with an example embodiment. As shown in Figure 2, the medication injection supervisor device 100 can record the dosage and time of each injection (202, 204, and 206) along with any warnings over a predetermined period of time. For example, for regular usage there can be up to four readings a day for thirty days. A user can have the ability to set up to four injection times and tolerances, these being the "scheduled" times the user is due to inject.

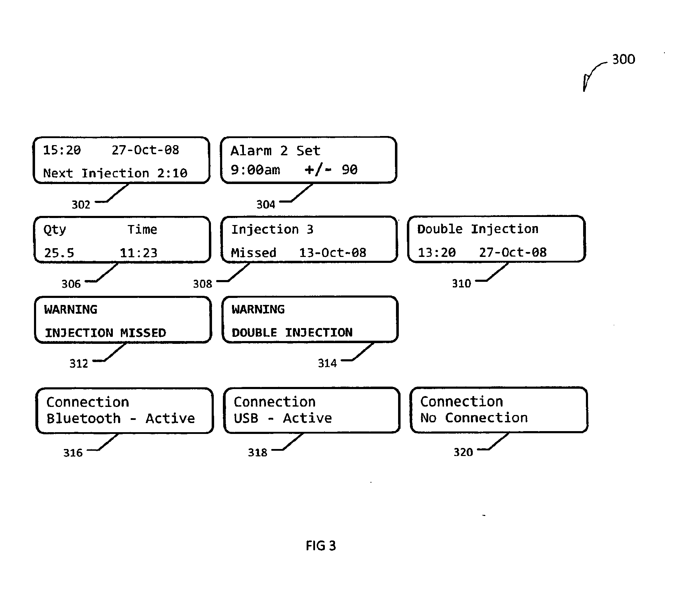

Figure 3 shows various messages that may appear on the display 132 of the medication injection supervisor device 100, in accordance with an example embodiment. The display 132, as shown in Figure 3, allows the user to step through the memory of recorded values directly from the medication injection supervisor device 100 as well as any warnings or messages. The medication injection supervisor device 100 has the ability to issue an alarm in both an auditory and a visual manner after the occurrence of one or more of the following: a missed injection, an injection dosage outside of the expected range, and a "double" injection. The medication injection supervisor device 100 can also check for and detect certain situations where a warning is to be provided. For example, the medication injection supervisor device 100 can warn when the cartridge of the medication pen 110 is low or the pen temperature has exceeded recommended safe range for the medication. The alarm may also be used to provide an alert, a reminder, or other scheduled event such as an "injection due" reminder or a reminder to change the needle after a given number of injections or uses.

The alarm features of the medication injection supervisor device 100 can address all of these points in a way that extends the basic ideal of a simple alarm. In this regard, the device can learn from a past usage pattern and can start to predict optimum injection times and suggest dosage. Thus, the device can monitor for dangerous usage, e.g. injecting a larger than recommended dosage or multiple dosages in a too-short period of time.

The device can also be comprised of a built-in SMS capability to automatically send injection usage to a "carer", to allow remote confirmation and monitoring for parents of young children as well as those looking after the elderly, to send general summary information concerning daily or weekly usage to nominated numbers, and to send realtime emergency SMS text messages to report "double dosage" to inform of a potential risk.

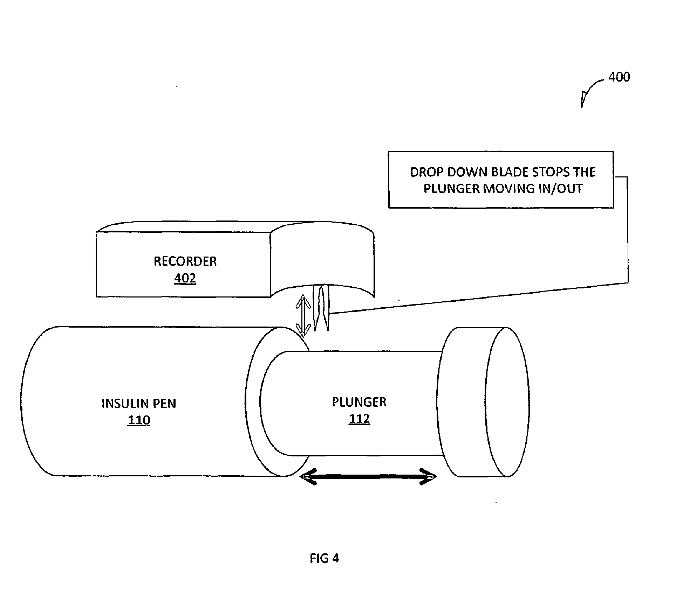

Figure 4 is a perspective view of a plunger break or locking pin device of the medication injection supervisor device 100, in accordance with an example embodiment. In some example embodiments, when the medication injection supervisor device 100 detects a "dangerous situation" (e.g. a too high a dose) not only can it generate an auditory and visual alarm, but it can also activate a locking pin as shown in Figure 4 that will prevent the user from pressing the plunger 112. This pin can be spring loaded and designed such that the user must dial a lower dose before the pin is released and the user allowed to proceed with the injection.

The medication injection supervisor device 100 may also include an interface to download data to and configure the device from a Personal Computer (PC). As the user builds up usage information over a number of weeks, this data is analyzed via separate software on the PC to produce trend information. This can be used to allow the user to "optimize" his medication usage, and is "fed back" into the pen to suggest the "expected" dosage and warn if the user is deviating significantly from it. This information can also be used by a medical practitioner to analyze any change in the user's condition.

The SMS functionality can allow a third party to be informed of any potentially dangerous situations concerning medication levels and generally, to allow monitoring whether the medication user is keeping to his recommended routine. This can be important when looking after young children and the elderly. The SMS feature can be extended further to allow text messages to be sent to the injection pen 110 to be displayed to the user on the display 132 should the usage data that has been downloaded indicate that a change in routine is required.

In some example embodiments, the user can step through the memory of recorded values, warnings, and messages directly from the device using the one ore more buttons 134. The device can have the ability to alarm in both an auditory and visual manner after the occurrence of missed injections, injection dosages being outside the expected range, and "double" injections. The device can also check for and detect certain situations where a warning is to be provided. For example, the device can warn when the cartridge is low or the pen temperature has exceeded its recommended safe range for medication.

In some example embodiments, the alarm may also be used to provide an alert for reminders or other scheduled events such as an "injection due" reminder and a reminder to change the injection pen's needle after a given number of injections or uses. Furthermore, the device can learn from a past usage pattern and can start predicting optimum injection times and suggest dosage amounts. Additionally, the device can monitor for dangerous usage, e.g. injecting a larger than recommended dosage or multiple dosages in a too-short period of time.

In some example embodiments, the device can facilitate remote monitoring of the patient's condition by sending information wirelessly. For example, the device can send information concerning injection usage to allow remote confirmation and monitoring to parents of young children and those who care for the elderly, send general summary information on daily or weekly usage to nominated numbers, and send instant emergency messages to report "double dosage" to inform of a potential risk.

In some example embodiments, the device can include an interface for downloading data to configure the device. As the user builds up usage information over a period of time, this data can be analyzed via separate software on a computing device to produce trend information. This information can be used to enable the user to optimize his medication usage, and can be fed back into the pen to suggest a dosage, and warn if the user's usage is deviating significantly.

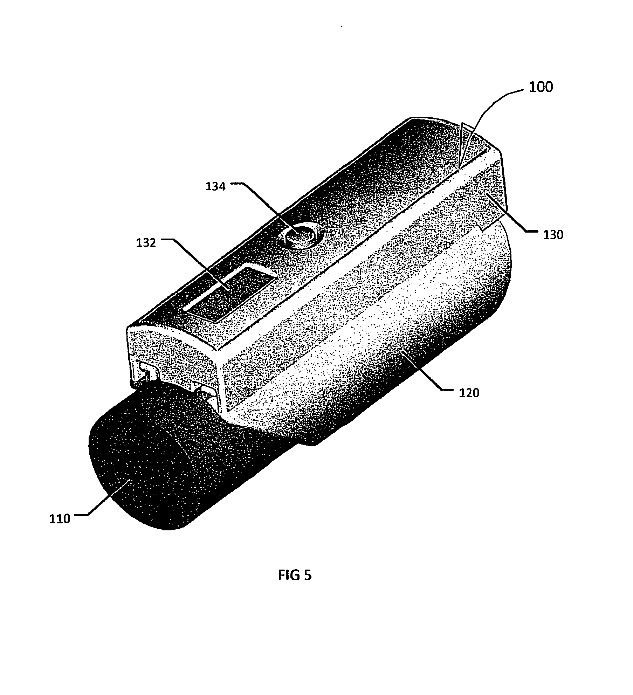

Figure 5 is a perspective view of the medication injection supervisor device 100 installed on the injection pen 110, in accordance with an example embodiment. As shown the medication injection supervisor device 100 can include the sleeve 120 and the universal header 130. The universal header 130 can, in turn, include the display 132 and the one or more buttons 134. The universal header 130 can be mounted on the sleeve 120 by sliding the universal header 130 on rails of the sleeve 120 designed to accommodate the universal header 130. When the universal header 130 is so mounted, the sleeve 120 squeezes the injection pen 110 holding the medication injection supervisor device tightly in place.

Thereafter, the medication injection supervisor device 100, according to an example embodiment, can assist in the control of medication injections by recording, monitoring, recommending, reporting, and protecting a user. The medication injection supervisor device 100 can be small enough so one can easily carry it with him wherever he goes as an attachment to a standard injection pen. In addition to providing a quick and easy method of recording his medication injection history, a user can be provided with a reminder or a warning if those reading were fed into the universal header 130 so that adjustments can be made in medication, exercise, or eating. This approach can provide someone with a chronic condition with a long-term view of his disease that will in turn help him control the disease and lead a healthier life without complications related thereto.

The medication injection supervisor device 100, according to an example embodiment, may require no user input because there is no need to confirm any action or to record any event. Thus, the medication injection supervisor device 100 can be fully automated. The medication injection supervisor device 100 can include only one button, which is used to turn on the display 132. The medication injection supervisor device 100 can record the dosage and time of each injection along with any warnings over a time period. It can have the ability to set a plurality of injection times and tolerances, which are the scheduled times the user is due to inject.

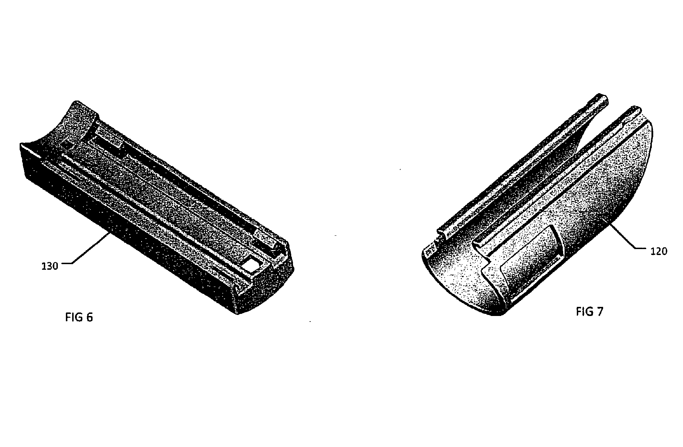

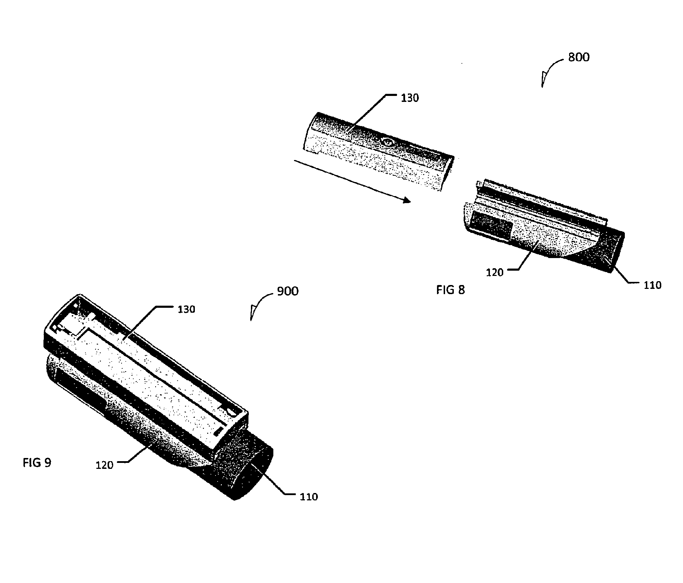

Figure 6 is an inverted view of the universal header 130, in accordance with an example embodiment. Figure 7 is the sleeve specific to each injection pen model, in accordance with an example embodiment. Figure 8 is the universal header 130 being mounted on a specific sleeve, in accordance with an example embodiment. Figure 9 is the universal header 130 mounted on a specific sleeve assembly, in accordance with an example embodiment. The medication injection supervisor device 100 can be mounted on a variety of prefabricated injection pens to record the time since last injection. This information assists the user of the prefabricated injection pen in maintaining optimum diabetic control.

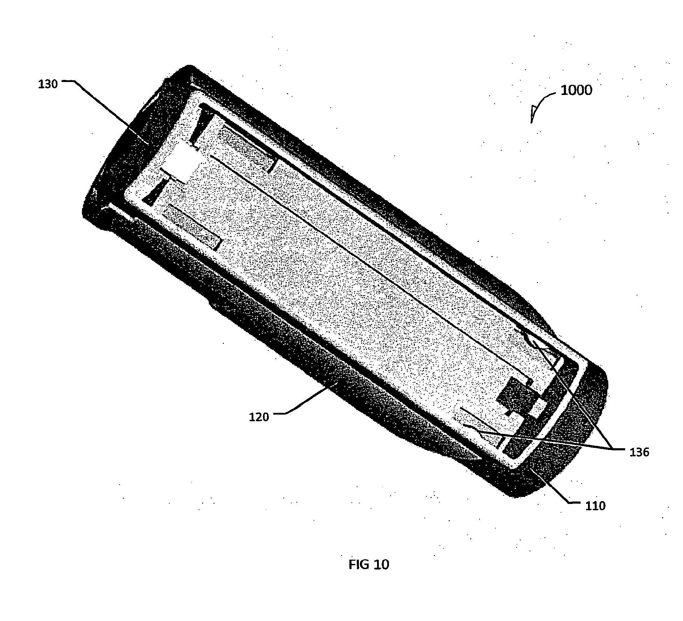

The medication injection supervisor device 100 automatically triggers whenever the user injects, and thus provides a simple and reliable means to note the elapsed time since the last injection. The prefabricated injection pens can be of various shapes, depending on their respective manufacturers. To accommodate these various shapes, a two-part attachment method is used. The first part is a sleeve designed to lock solidly and robustly on the particular injection pen. The second part is a universal header unit attached to the sleeve that houses the electronics. Figure 10 is an empty universal header and specific sleeve assembly mounted on a specific injection pen model with the locking indents visible 136, in accordance with an example embodiment.

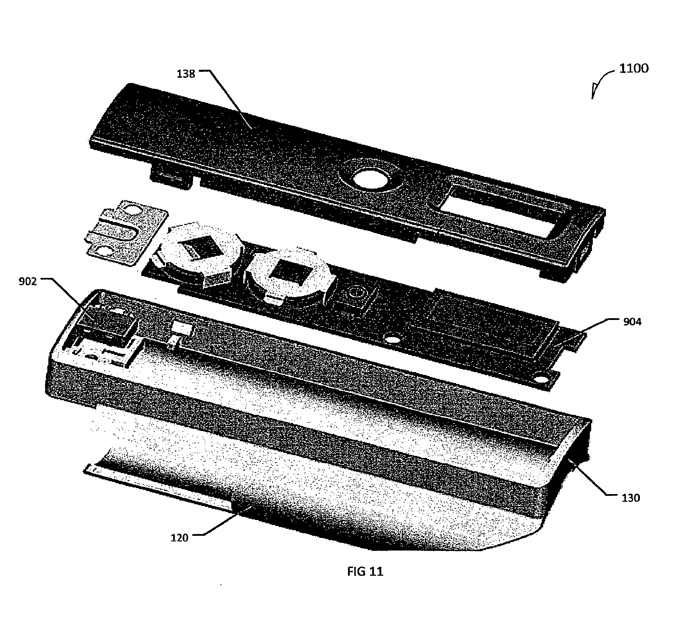

Figure 11 is an exploded view of the medication injection supervisor device 100, in accordance with an example embodiment. As shown, the medication injection supervisor device 100 can include a cover plate 138, an electronic assembly 904, a sensor 902, a medication pen, the universal header 130, and the sleeve 120. In some example embodiments, when the medication injection supervisor device 100 is mounted on an injection pen 110, it positions the sensor 902 over the edge of the plunger (not shown) of the injection pen 110. In most injection pens, users extend the plunger to dial a dosage. When the plunger is extended, the sensor 902 (e.g. a micro switch) is toggled to the open state. When the sensor 902 is toggled to the open state, the timer is reset to zero to indicate that the injection has started.

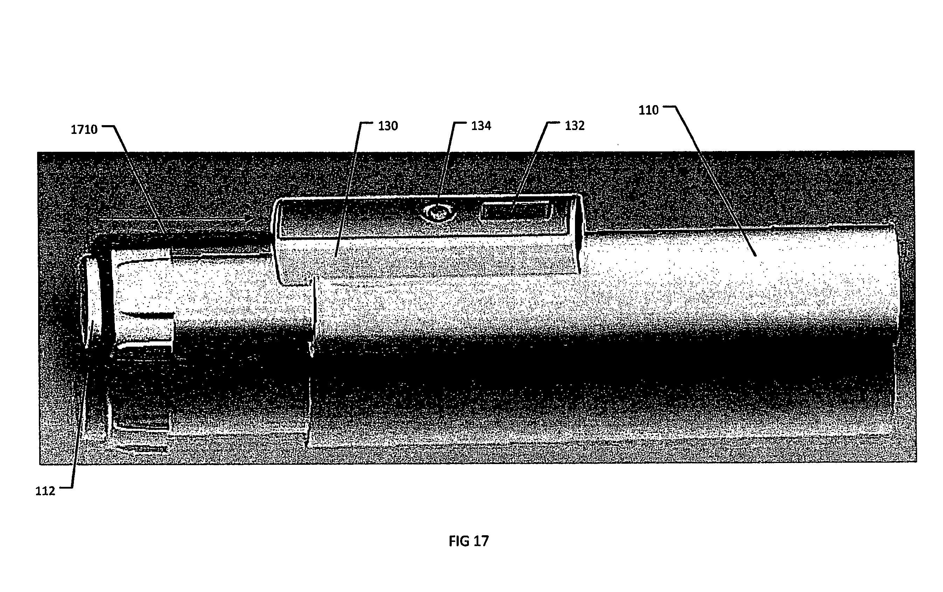

When the plunger is pressed to complete the injection, the sensor 902 is closed and the timer starts counting. In some example embodiments, a sound-generating unit (not shown) and/or a vibrational unit (not shown) can be installed to alert the user. It will be understood that some injection pens may not use a plunger to facilitate injections. Therefore, other techniques can be used to determine the occurrence of an injection and / or the dosage. For example, a micro optics and imaging system, as described below with reference to Figure 17 can be used to determine the occurrence of an injection and / or the dosage.

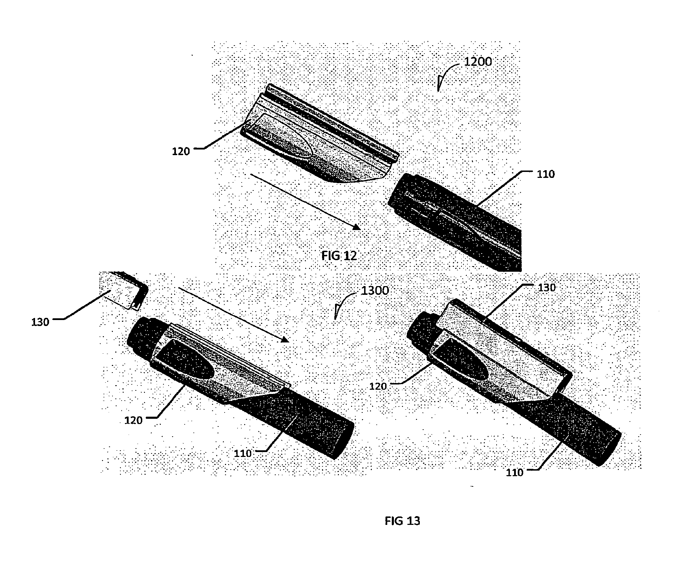

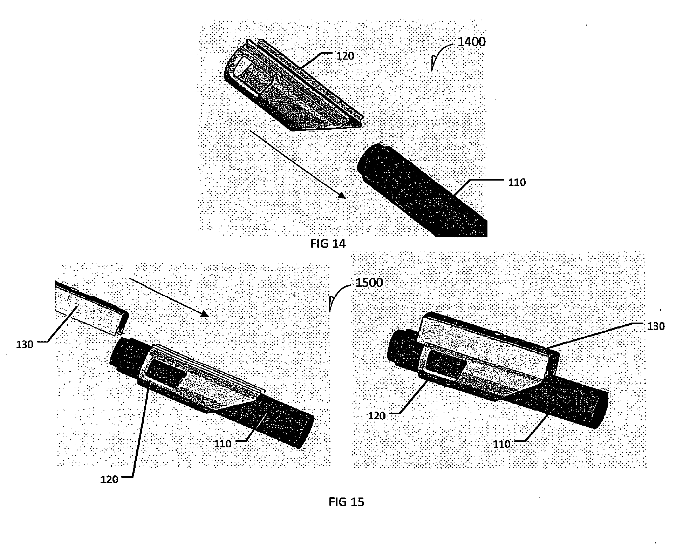

Figure 12 shows a sleeve being mounted on a Sanofi Aventis Lantus SoloStar medication pen, in accordance with an example embodiment. Figure 13 shows a universal header being installed on a sleeve mounted on a Sanofi Aventis Lantus SoloStar medication pen, in accordance with an example embodiment. Figure 14 shows a sleeve being mounted on a Novo Nordisk Flexpen medication pen, in accordance with an example embodiment. Figure 15 shows a universal header being installed on a sleeve mounted on a Novo Nordisk FlexPen medication pen, in accordance with an example embodiment.

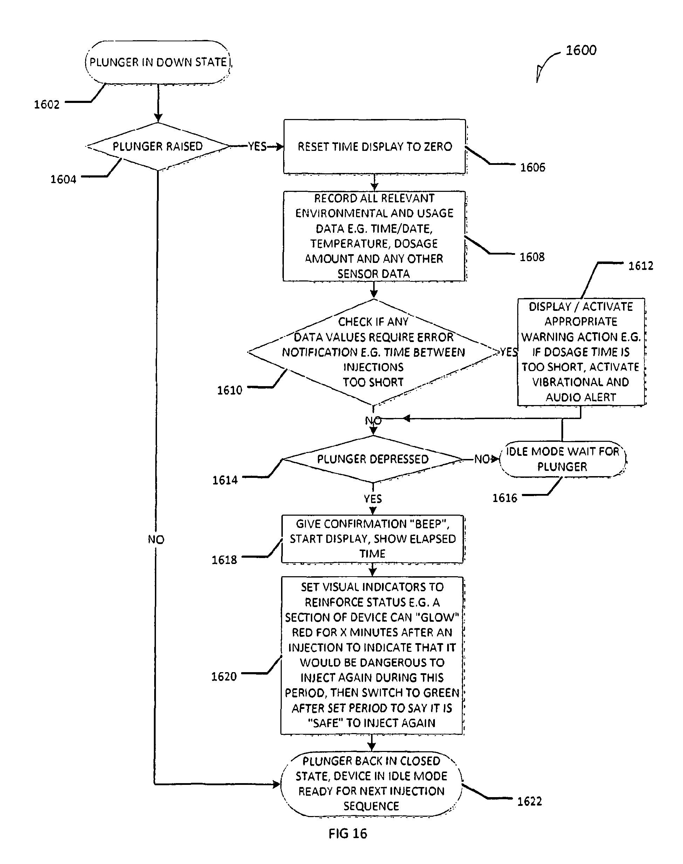

Figure 16 is a flow diagram showing a method 1600 for supervising injections using the medication injection supervisor device, in accordance with an example embodiment. The method can commence at operation 1602 when the plunger 112 is in the down state. At decision block 1604 it can be determined whether or not the plunger 112 is raised. If the plunger 112 is raised at operation 1602 to dial a dosage, the method 1600 can proceeds to operation 1606 where the time display is reset to zero, thus starting the time count from the last injection. At operation 1608, relevant environmental and usage data can be recorded. The relevant environmental and usage data can include time, date, temperature, dosage amount, and other sensor data.

At decision block 1610, it is determined based on the comparison of the recorded environmental and usage data to predetermined optimal values, whether or not an error notification needs to be issued. For example, if the time between injections is too short, an error can be communicated to the user. Thus, if at decision block 1610 it is determined that an error notification needs to be made, the method 1600 proceeds to operation 1612 where the error is displayed via the display 132. In some example embodiments, other appropriate warning actions can be taken. For example, if the time interval between two consecutive injections is too short, vibrational and/or audio alerts can be activated.

If, on the other hand, it is determined at decision block 1610 that no error notification needs to be made, the error is not communicated. In either case, the method 1600 can proceed and at decision block 1614 it can be determined whether the plunger 112 is depressed. If the plunger 112 is not depressed, the method 1600 remains in the idle mode waiting for the plunger 112 to be depressed. Once the plunger 112 is depressed, the method 1600 can proceed to operation 1618 where the display 132 can start measuring time from the last injection by showing the elapsed time. Additionally, a confirmation "beep" can be provided.

At operation 1620, visual indicators can be set to reinforce the status. For example, a section of the device can "glow" red for a predetermined time interval after the injection to indicate that it would be dangerous to inject again during this period. Once the predetermined time interval has elapsed, the "glow" can become green indicating that it is safe to inject again. Additionally, if the elapsed time is further exceeded past a point where another injection is expected but has not occurred the "glow" can change to yellow.

Figure 17 shows a readable plunger subsystem, in accordance with an example embodiment. As shown in Figure 17, a secondary digitally readable plunger subsystem can be physically attached to the plunger head via a pen specific clip 1710 so that this secondary plunger moves in parallel with the injection pen plunger 112. As the secondary plunger slides in and out of the universal header 130, an electronics assembly inside the header 130 can read linear positions of the secondary plunger and thus infer the dosage of the medication. During the motion of the secondary plunger, the display 132 can show the user the inferred dosage for confirmation. The readings taken by the electronics assembly can be also used to time the occurrences of injections.

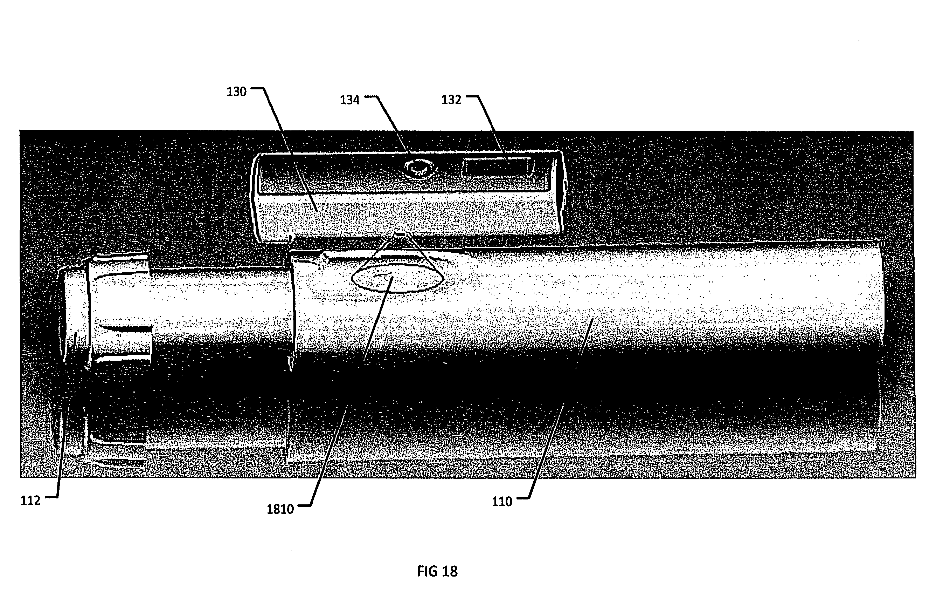

Figure 18 shows a micro optics and imaging system, in accordance with an example embodiment. As shown in Figure 18, the micro optics and imaging system can be incorporated in the universal header 130. The sleeve (not shown) can be designed to ensure that the universal header 130 is positioned over the physical dosage dial of the injection pen 110. When the sensor (not shown) is triggered to indicate that an injection is about to start, an imaging system 1810 can be activated and an image recognition performed by reading the maximum dial value which is then stored in the memory of the medication injection supervisor device 100. During the imaging sequence, the raw data can also be displayed on the display 132 to allow the user to the actual dial value.

Thus, example embodiments of a medication injection supervisor device have been described. Although embodiments have been described with reference to specific example embodiments, it will be evident that various modifications and changes may be made to these embodiments without departing from the scope of the system and method described herein. Accordingly, the specification and drawings are to be regarded in an illustrative rather than a restrictive sense.

## Claims

1. A medication injection supervisor device (100) comprising:
a universal header (130) mountable on a prefabricated injection pen (110), and
an electronics assembly (904) housed by the universal header (130) to record time,
the medication injection supervisor device (100) being automatically triggerable whenever a user injects medication to provide a reliable means to note the time of or time elapsed since the last injection **characterised in that** the medication injection supervisor device (100) further comprises a sleeve (120) adapted to the prefabricated injection pen (110), the universal header (130) being attachable to the sleeve (120) for mounting the medication injection supervisor device (100) on the prefabricated injection pen (110).

2. A medication injection supervisor device (100) as claimed in Claim 1 wherein the universal header (130) is adapted to rigidly lock onto the sleeve (120).

3. A medication injection supervisor device (100) as claimed in Claim 1 or Claim 2 wherein the sleeve (120) is adapted to rigidly lock onto the prefabricated injection pen (110).

4. A medication injection supervisor device (100) as claimed in any of Claims 1 to 3 wherein the universal header (130) and the sleeve (120) comprise a two-part attachment, the sleeve (120) being customised to fit a specific model of prefabricated injection pen (110).

5. A medication injection supervisor device (100) as claimed in any of Claims 1 to 4 further comprising a sensor (902) communicatively coupled to the electronics assembly (904) to detect an injection automatically and a display (132) to display injection data.

6. A medication injection supervisor device (100) as claimed in Claim 5 further comprising one or more buttons (134) to allow manipulation and display of the injection data.

7. A medication injection supervisor device (100) as claimed in any of Claims 1 to 6 wherein the medication injection supervisor device (100) is automatically triggered upon depression of a plunger (112) on the injection pen (110).

8. A medication injection supervisor device (100) as claimed in any of Claims 1 to 7 further comprising one or more sensors (902) to detect environmental and usage data, the environmental and usage data including a time of injection, a temperature at the time of injection and a dosage.

9. A medication injection supervisor device (100) as claimed in any of Claims 1 to 8 wherein the electronics assembly (904) is adapted to keep records related to a prescribed injection schedule.

10. A medication injection supervisor device (100) as claimed in Claim 9 wherein the records related to a prescribed injection schedule comprise dosage and the time of each injection.

11. A medication injection supervisor device (100) as claimed in any of Claims 1 to 10 further comprising an alarm generator.

12. A medication injection supervisor device (100) as claimed in Claim 11 wherein the alarm generator comprises a visual alarm, an audio alarm, a vibrational alarm or an SMS message.

13. A medication injection supervisor device (100) as claimed in any of Claims 1 to 12 wherein the electronics assembly (904) is communicable wirelessly to communicate information and facilitate remote monitoring of a patient's condition via one or more wireless protocols.

14. A medication injection supervisor device (100) as claimed in any of Claims 1 to 13 further comprising a locking pin to prevent an injection by blocking an injection mechanism (112) of the injection pen (110) upon determination that the injection is to create an overdose condition.

## Patentansprüche

1. Eine Vorrichtung (100) zur Überwachung der Injektion von Medikamenten, umfassend:
einen Universalkopf (130), der auf einem vorfabrizierten Injektionsstift (110) montierbar ist, und
eine von dem Universalkopf (130) untergebrachte Elektronikanordnung (904) zum Aufzeichnen von Zeit,
wobei die Vorrichtung (100) zur Überwachung der Injektion von Medikamenten jedes Mal, wenn ein Benutzer ein Medikament injiziert, automatisch auslösbar ist, um ein verlässliches Mittel bereitzustellen, um den Zeitpunkt der letzten Injektion oder die Zeit, die seit dieser verstrichen ist, zu vermerken, **dadurch gekennzeichnet, dass** die Vorrichtung (100) zur Überwachung der Injektion von Medikamenten ferner eine Hülse (120) umfasst, die an den vorfabrizierten Injektionsstift (110) angepasst ist, wobei der Universalkopf (130) an der Hülse (120) anbringbar ist, um die Vorrichtung (100) zur Überwachung der Injektion von Medikamenten auf dem vorfabrizierten Injektionsstift (110) zu montieren.

2. Vorrichtung (100) zur Überwachung der Injektion von Medikamenten gemäß Anspruch 1, wobei der Universalkopf (130) angepasst ist, um auf der Hülse (120) unbeweglich einzurasten.

3. Vorrichtung (100) zur Überwachung der Injektion von Medikamenten gemäß Anspruch 1 oder Anspruch 2, wobei die Hülse (120) angepasst ist, um auf dem vorfabrizierten Injektionsstift (110) unbeweglich einzurasten.

4. Vorrichtung (100) zur Überwachung der Injektion von Medikamenten gemäß einem der Ansprüche 1 bis 3, wobei der Universalkopf (130) und die Hülse (120) ein zweiteiliges Anbauteil umfassen, wobei die Hülse (120) maßgefertigt ist, um zu einem spezifischen Modell eines vorfabrizierten Injektionsstifts (110) zu passen.

5. Vorrichtung (100) zur Überwachung der Injektion von Medikamenten gemäß einem der Ansprüche 1 bis 4, ferner umfassend einen Sensor (902), der mit der Elektronikanordnung (904) kommunikativ verbunden ist, um automatisch eine Injektion zu erfassen, und eine Anzeige (132), um Injektionsdaten anzuzeigen.

6. Vorrichtung (100) zur Überwachung der Injektion von Medikamenten gemäß Anspruch 5, ferner umfassend eine oder mehrere Tasten (134), um die Manipulation und Anzeige der Injektionsdaten zu ermöglichen.

7. Vorrichtung (100) zur Überwachung der Injektion von Medikamenten gemäß einem der Ansprüche 1 bis 6, wobei die Vorrichtung (100) zur Überwachung der Injektion von Medikamenten beim Drücken eines Kolbens (112) auf dem Injektionsstift (110) automatisch ausgelöst wird.

8. Vorrichtung (100) zur Überwachung der Injektion von Medikamenten gemäß einem der Ansprüche 1 bis 7, ferner umfassend einen oder mehrere Sensoren (902) zum Erfassen von Umgebungs- und Nutzungsdaten, wobei die Umgebungs- und Nutzungsdaten einen Zeitpunkt der Injektion, eine Temperatur zum Zeitpunkt der Injektion und eine Dosierung einschließen.

9. Vorrichtung (100) zur Überwachung der Injektion von Medikamenten gemäß einem der Ansprüche 1 bis 8, wobei die Elektronikanordnung (904) angepasst ist, um Aufzeichnungen hinsichtlich eines vorgeschriebenen Injektionsplans zu führen.

10. Vorrichtung (100) zur Überwachung der Injektion von Medikamenten gemäß Anspruch 9, wobei die Aufzeichnungen hinsichtlich eines vorgeschriebenen Injektionsplans die Dosierung und den Zeitpunkt jeder Injektion umfassen.

11. Vorrichtung (100) zur Überwachung der Injektion von Medikamenten gemäß einem der Ansprüche 1 bis 10, ferner umfassend einen Alarmgeber.

12. Vorrichtung (100) zur Überwachung der Injektion von Medikamenten gemäß Anspruch 11, wobei der Alarmgeber einen sichtbaren Alarm, einen hörbaren Alarm, einen Schwingungsalarm oder eine SMS-Nachricht umfasst.

13. Vorrichtung (100) zur Überwachung der Injektion von Medikamenten gemäß einem der Ansprüche 1 bis 12, wobei die Elektronikanordnung (904) drahtlos kommunizierfähig ist, um Informationen zu kommunizieren und die Fernüberwachung des Zustandes eines Patienten über eines oder mehrere drahtlose Protokolle zu erleichtern.

14. Vorrichtung (100) zur Überwachung der Injektion von Medikamenten gemäß einem der Ansprüche 1 bis 13, ferner umfassend einen Arretierbolzen, um eine Injektion zu verhindern, indem ein Injektionsmechanismus (112) des Injektionsstifts (110) nach Bestimmung, dass die Injektion einen Überdosiszustand schaffen würde, gesperrt wird.

## Revendications

1. Un dispositif de surveillance d'injection de médicament (100) comprenant :
un collecteur universel (130) pouvant être monté sur un stylo à injection préfabriqué (110), et
un assemblage d'électronique (904) logé dans le collecteur universel (130) pour enregistrer le temps,
le dispositif de surveillance d'injection de médicament (100) pouvant être automatiquement déclenché chaque fois qu'un utilisateur injecte le médicament afin de fournir un moyen fiable de noter l'heure de ou le temps écoulé depuis la dernière injection **caractérisé en ce que** le dispositif de surveillance d'injection de médicament (100) comprend en outre un manchon (120) adapté au stylo à injection préfabriqué (110), le collecteur universel (130) pouvant être attaché au manchon (120) afin de monter le dispositif de surveillance d'injection de médicament (100) sur le stylo à injection préfabriqué (110).

2. Un dispositif de surveillance d'injection de médicament (100) tel que revendiqué dans la revendication 1 dans lequel le collecteur universel (130) est adapté afin de se verrouiller de façon rigide sur le manchon (120).

3. Un dispositif de surveillance d'injection de médicament (100) tel que revendiqué dans la revendication 1 ou la revendication 2 dans lequel le manchon (120) est adapté pour se verrouiller de façon rigide sur le stylo à injection préfabriqué (110).

4. Un dispositif de surveillance d'injection de médicament (100) tel que revendiqué dans n'importe lesquelles des revendications 1 à 3 dans lequel le collecteur universel (130) et le manchon (120) constituent un attachement en deux parties, le manchon (120) étant personnalisé pour correspondre à un modèle spécifique de stylo à injection préfabriqué (110).

5. Un dispositif de surveillance d'injection de médicament (100) tel que revendiqué dans n'importe lesquelles des revendications 1 à 4 comprenant en outre un capteur (902) couplé de façon à communiquer avec l'assemblage d'électronique (904) afin de détecter automatiquement une injection et un affichage (132) afin d'afficher des données d'injection.

6. Un dispositif de surveillance d'injection de médicament (100) tel que revendiqué dans la revendication 5 comprenant en outre un ou plusieurs boutons (134) pour permettre la manipulation et l'affichage des données d'injection.

7. Un dispositif de surveillance d'injection de médicament (100) tel que revendiqué dans n'importe lesquelles des revendications 1 à 6 dans lequel le dispositif de surveillance d'injection de médicament (100) est automatiquement déclenché lorsqu'un plongeur (112) est poussé sur le stylo à injection (110).

8. Un dispositif de surveillance d'injection de médicament (100) tel que revendiqué dans n'importe lesquelles des revendications 1 à 7 comprenant en outre un ou plusieurs capteurs (902) pour détecter des données environnementales et d'utilisation, les données environnementales et d'utilisation incluant une heure d'injection, une température à l'heure de l'injection et une dose.

9. Un dispositif de surveillance d'injection de médicament (100) tel que revendiqué dans n'importe lesquelles des revendications 1 à 8 dans lequel l'assemblage électronique (904) est adapté pour conserver des enregistrements relatifs à un programme d'injections prescrit.

10. Un dispositif de surveillance d'injection de médicament (100) tel que revendiqué dans la revendication 9 dans lequel les enregistrements relatifs à un programme d'injections prescrit comprennent la dose et l'heure de chaque injection.

11. Un dispositif de surveillance d'injection de médicament (100) tel que revendiqué dans n'importe lesquelles des revendications 1 à 10 comprenant en outre un générateur d'alarme.

12. Un dispositif de surveillance d'injection de médicament (100) tel que revendiqué dans la revendication 11 dans lequel le générateur d'alarme comprend une alarme visuelle, une alarme sonore, une alarme vibratoire ou un message SMS.

13. Un dispositif de surveillance d'injection de médicament (100) tel que revendiqué dans n'importe lesquelles des revendications 1 à 12 dans lequel l'assemblage d'électronique (904) peut communiquer sans fil afin de communiquer des informations et de faciliter la surveillance à distance de l'état d'un patient grâce à un ou plusieurs protocoles sans fil.

14. Un dispositif de surveillance d'injection de médicament (100) tel que revendiqué dans n'importe lesquelles des revendications 1 à 13 comprenant en outre une broche de verrouillage pour empêcher une injection en bloquant un mécanisme d'injection (112) du stylo à injection (110) dès qu'il est déterminé que l'injection va créer un état d'overdose.
